(19) Europäisches Patentamt

European Patent Office

Office européen des brevets

(11) **EP 0 800 455 B1**

(12) **EUROPEAN PATENT SPECIFICATION**

(45) Date of publication and mention
of the grant of the patent:
**27.02.2002 Bulletin 2002/09**

(21) Application number: **95944361.5**

(22) Date of filing: **21.12.1995**

(51) Int Cl.[7]: **B32B 27/08**, A61L 15/00

(86) International application number:
**PCT/US95/16696**

(87) International publication number:
**WO 96/20831 (11.07.1996 Gazette 1996/31)**

(54) **A WATER-FLUSHABLE FILM**

MIT WASSER WEGSPÜLBARER FILM

FILM EVACUABLE DANS L'EAU

(84) Designated Contracting States:
**BE DE ES FR GB IT NL SE**

(30) Priority: **30.12.1994 US 367654**

(43) Date of publication of application:
**15.10.1997 Bulletin 1997/42**

(73) Proprietor: **Kimberly-Clark Worldwide, Inc.
Neenah, WI 54956 (US)**

(72) Inventors:
• **LARSON, Jennifer, Cappel
Oshkosh, WI 54904 (US)**
• **POMPLUN, William, Seal
Neenah, WI 54956 (US)**

(74) Representative:
**Diehl, Hermann, Dr. Dipl.-Phys. et al
DIEHL, GLÄSER, HILTL & PARTNER
Patentanwälte Augustenstrasse 46
80333 München (DE)**

(56) References cited:
| | |
|---|---|
| **EP-A- 0 010 171** | **EP-A- 0 032 244** |
| **EP-A- 0 142 950** | **EP-A- 0 226 439** |
| **EP-A- 0 514 137** | **EP-A- 0 525 245** |
| **EP-A- 0 589 437** | **WO-A-92/01556** |
| **WO-A-92/15454** | **WO-A-94/00293** |
| **US-A- 4 372 311** | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

Background of the Invention

Field of the Invention

The present invention relates to a flexible polymeric film which exhibits improved flushability properties when immersed in water. The film may be used in a disposable absorbent product to impart improved flushability properties to the product after the product's use.

Description of the Related Art

Disposable absorbent products currently find widespread use in many applications. For example, in the infant and child care areas, diapers and training pants have generally replaced reusable cloth absorbent articles. Other typical disposable absorbent products include feminine care products such as sanitary napkins or tampons, adult incontinence products, and health care products such as surgical drapes or wound dressings. A typical disposable absorbent product generally comprises a composite structure including a topsheet, a backsheet, and an absorbent structure between the topsheet and backsheet. These products usually include some type of fastening system for fitting the product onto the wearer.

Disposable absorbent products are typically subjected to one or more liquid insults, such as of water, urine, menses, or blood, during use. As such, the outer cover backsheet materials of the disposable absorbent products are typically made of liquid-insoluble and liquid impermeable materials, such as polypropylene films, that exhibit a sufficient strength and handling capability so that the disposable absorbent product retains its integrity during use by a wearer and does not allow leakage of the liquid insulting the product.

Although current disposable baby diapers and other disposable absorbent products have been generally accepted by the public, these products still have need of improvement in specific areas. For example, many disposable absorbent products can be difficult to dispose of into an aqueous environment. For example, attempts to flush many disposable absorbent products down a toilet into a sewage system typically lead to blockage of the toilet or pipes connecting the toilet to the sewage system. In particular, the outer cover materials typically used in the disposable absorbent products generally do not disintegrate or disperse when flushed down a toilet so that the disposable absorbent product cannot be disposed of in this way. If the outer cover materials are made very thin in order to reduce the overall bulk of the disposable absorbent product so as to reduce the likelihood of blockage of a toilet or a sewage pipe, then the outer cover material typically will not exhibit sufficient strength to prevent tearing or ripping as the outer cover material is subjected to the stresses of normal use by a wearer.

As such, there is a need for new materials that may be used in disposable absorbent products that generally retain their integrity. and strength during use, but after such use, the materials may be disposed of into an aqueous environment. For example, the disposable absorbent product may be easily and efficiently disposed of by flushing the disposable absorbent product down a toilet. Such a disposable absorbent product would then be capable of being degraded by a liquid sewage system as compared to having to be disposed of into a landfill or other solid waste disposal system.

From WO92/02199 a composite material for use in a disposable product, such as a nappy/diaper, is known which is flushable and biodegradable. The composite material comprises an outer soluble layer which supports a disintegratable and dispersable liquid absorbent layer and a liquid impervious barrier interposed between the soluble layer and the absorbent layer.

From WO 92/01556 a layer product is known comprising a thin layer of an insoluble polymer superimposed on a thicker layer of a soluble polymer. Both polymers are conveniently polyvinyl alcohol.

WO 92/15454 discloses compostable polymeric sheets of biodegradable or environmentally degradable polymers. The compostable polymeric sheets comprise an intermediate film of water soluble polymer interposed between a water-insoluble top and bottom layer which bonds the top and bottom layers to each other.

EP 0,010,171 A1 discloses a composite film consisting of a first water-resilient layer, a second layer attached to said first layer, said second layer consisting of a water-soluble, natural or synthetic polymer, and a third disintegratable layer bonded to said first and second layer made from a water-insoluble, hydrophilic, natural or synthetic polymer.

Summary of the Invention

The present invention concerns a film that substantially disperses when contacted with an excess amount of water. Such a film may be used in a disposable absorbent product to increase the flushability of the product into a liquid sewage system.

One aspect of the present invention concerns a film comprising film comprising a liquid barrier layer that has a thickness between 2 to 15 micrometers attached to a water-dispersible layer that has a thickness between 10 to 100 micrometers and comprises 55 to 95 wt.-% polyethylene oxide and 45 to 5 wt.-% poly(ethylene-co-acrylic acid), based on the total amount of polyethylene oxide and poly(ethylene-co-acrylic acid) present in said water-dispersible layer, and wherein the film has an average density greater than about 1 gram per cubic centimeter.

In another aspect, the present invention concerns a disposable absorbent product comprising a liquid-permeable topsheet, a backsheet attached to the liquid-permeable topsheet, and an absorbent structure positioned between the topsheet and the backsheet, wherein the backsheet comprises the film of the present invention.

Brief Description of the Drawings

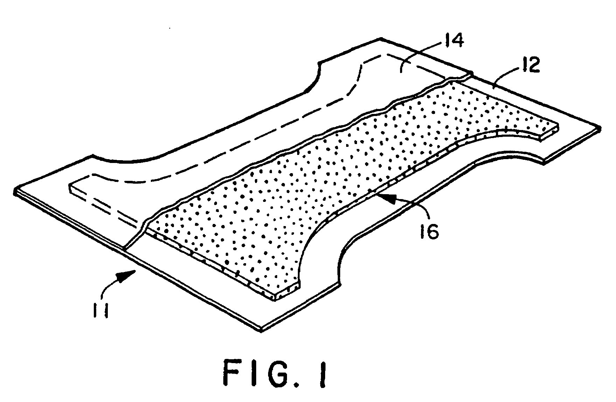

Fig. 1 represents a disposable absorbent product according to the present invention.

Detailed Description of the Preferred Embodiments

The present invention, in one aspect, concerns a film material that exhibits desired water-dispersibility properties and is prepared from extrudable compositions. The film generally comprises a barrier layer and a water-dispersible layer.

As used herein, the term "water-dispersible layer" is meant to refer to a layer of the film of the present invention that when placed in an aqueous environment will, with sufficient time, break apart into smaller pieces. As a result, the water-dispersible layer once dispersed may be more advantageously processable in recycling processes or flushable in, for example, septic and municipal sewage treatment systems. If desired; the dispersal of the water-dispersible layer may be hastened by the use of agitation and/or certain triggering means. The actual amount of time needed for dispersal of the water-dispersible layer will typically depend at least in part upon the particular end-use design criteria. Typically, the water-dispersible layer will be fully dispersed within the aqueous environment into which the water-dispersible layer has been placed within 60 minutes, suitably within 15 minutes, more suitably within 5 minutes, and most suitably within 30 seconds.

The water-dispersible layer may also comprise an amount of a non-water-dispersible polymer in order to improve the strength and handling properties of the layer as'long as the layer still exhibits the desired water-dispersible properties. The use of the non-water-dispersible polymer may allow for the use of a more water-sensitive polymer as the water-dispersible polymer which, if used alone, would have very poor handling characteristics and would be liable to crack or tear during use.

The water-dispersible layer of the present invention is prepared from a mixture comprising polyethylene oxide and poly(ethylene-co-acrylic acid). In such a mixture, the polyethylene oxide is present in an amount from 55 weight percent to 95 weight percent and the poly(ethylene-co-acrylic acid) is present in an amount from 45 weight percent to 5 weight percent, wherein all weight percents are based on the total amount of polyethylene oxide and poly(ethylene-co-acrylic acid) present in the water-dispersible layer.

The water-dispersible layer may generally be prepared by known processes such as thermal extrusion processes or solvent casting processes.

The water-dispersible layer should be present in the film in an amount effective to achieve the desired strength and water-dispersibility properties. As such, the water-dispersible layer should be present in the film in less than an excessive amount so that the film exhibits the desired water-dispersible properties within a desired time period. In addition, the water-dispersible layer should be present in the film in more than a minimal amount so that the film exhibits the desired strength properties. The desired amount of the water-dispersible layer present in the film can be quantified in one embodiment by the thickness of the water-dispersible layer.

The water-dispersible layer is therefore present in a film of the present invention in an amount such that the thickness of the water-dispersible layer is between 10 micrometers and 100 micrometers, suitably between about 10 to 50 micrometers, more suitably between 10 to 30 micrometers, and most suitably between 10 to 20 micrometers.

As used herein, the term "barrier layer" is meant to refer to a layer of the film of the present invention that is liquid impermeable such that the barrier layer will effectively not allow any liquid to pass through the barrier layer during an amount of time in which a liquid is in contact with the barrier layer.

In particular, as used herein, the liquid impermeability of a material may be measured according to the Film Liquid Transient Exposure Test as described in the Test Method section herein. In general, a material or layer will be considered to be a barrier layer if it passes the Film Liquid Transient Exposure Test in that no aqueous liquid is observed to pass through the layer being evaluated during a 60 minute period. Alternatively, a material or layer will be considered to not be a barrier layer if it does not pass the Film Liquid Transient Exposure Test in that aqueous liquid is observed to pass through the layer being evaluated during a 60 minute period.

The barrier layer is typically prepared from a polymer that in the form of a film is liquid impermeable. Examples of polymers useful in preparing the barrier layer useful in the present invention include acrylic acid copolymer, polylactic acid, maleic anhydride modified polyethylene, poly(ethylene-co-acrylic acid), polycaprolactone, polyester, polyhydroxyalkanoates such as polyhydroxybutyrate/hydroxyvalerate, polyhydroxybutyrate, and polyhydroxyvalerate, and copolymers and mixtures thereof.

The barrier layer may also comprise an amount of a water-dispersible polymer in order to improve the decomposition properties of the barrier layer as long as the barrier layer still exhibits the desired water-impermeability properties.

In one embodiment of the present invention, the barrier layer is desirably biodegradable. Such biodegradability of the barrier film will assist in decomposition of the barrier layer when the film is disposed of. Examples of biodegradable barrier layer include polylactic acid, polycaprolactone, certain synthetic polyesters, polyhydroxyalkanoates such as polyhydroxybutyrate/hydroxyvalerate, polyhydroxybutyrate, and polyhydroxyvalerate, and copolymers and mixtures thereof.

The barrier layer should be present in the film in an amount effective to achieve the desired strength and water-dispersibility properties. As such, the barrier layer should be present in the film in less than an excessive amount so that the film exhibits the desired water-dispersible properties within a desired time period. In addition, the barrier layer should be present in the film in more than a minimal amount so that the film exhibits the desired strength properties. The desired amount of the barrier layer present in the film can be quantified in one embodiment by the thickness of the barrier layer.

The barrier layer is therefore present in a film of the present invention in an amount such that the thickness of the barrier layer is between 2 micrometers to 15 micrometers, suitably between 2 to 10 micrometers, more suitably between 2 to 7 micrometers, and most suitably between 2 to 5 micrometers.

A film of the present invention generally has the structure of a continuous sheet of material, with no identifiable, individual fibers or the like. The film comprises at least two layers, with one layer being a barrier layer and a second layer being a water-dispersible layer. The barrier layer will be attached to the water-dispersible layer with an adhesive or suitably by the cohesion between the layers. The film may also comprise a third layer, such as a spunbond fibrous material, attached to the water-dispersible layer, that helps to prevent premature dispersibility of the water-dispersible layer which may be caused, for example, by spilled water or wet hands of someone handling the film.

Spunbond fibrous materials are generally formed by extruding a molten thermoplastic material as filaments from a plurality of fine, usually circular, capillaries, in a spinnerette with the diameter of the extruded filaments then being rapidly reduced, for example, by non-eductive or eductive fluid-drawing or other well-known spunbonding mechanisms. The production of spunbond nonwoven webs is illustrated in patents such as US-A-4,340,563 to Appel et al.; US-A-3,802,817 to Matsuki et al.; US-A-3,692,618 to Dorschner et al.; US-A-3,338,992 and US-A-3,341,394 to Kinney; US-A-3,276,944 to Levy; US-A-3,502,538 to Peterson; US-A-3,502,763 to Hartman; US-A-3,542,615 to Dobo et al.; and CA-B-803,714 to Harmon.

Films are known to be able to be prepared by a variety of processes such as, for example, extrusion processes and casting processes.

In general, the film of the present invention may be prepared from two separate thermoplastic compositions wherein one composition comprises the polymer to be used to prepare the barrier layer and a second composition comprises the polymer to be used to prepare the water-dispersible layer. As used herein, the term "thermoplastic" is meant to describe a material that softens when exposed to heat and which substantially returns to its original condition when cooled to room temperature.

While the principal components of the film of the present invention have been described in the foregoing, it can additionally include other components not adversely effecting the desired water-dispersibility properties of the film. Exemplary materials which could be used as additional components include, without limitation, pigments, antioxidants, plasticizers, stabilizers, surfactants, waxes, flow promoters, solid solvents, particulates, and materials added to enhance processability of the film.

It is desirable that the film of the present invention exhibit desirable water-dispersibility properties when immersed in an excess of water for a period of time.

As used herein, the term "immersed" is intended to represent that a material is substantially completely submerged into or otherwise substantially completely surrounded by the water. As used herein, the term "excess of water" is intended to represent that the amount of water into which a material is immersed is effective to substantially completely surround the material being immersed and wherein any amount of water absorbed by the material being immersed represents an insubstantial amount of the total amount of water being used. As such, the amount of water used to immerse the material must be sufficiently large so as to take into consideration the amount of water that may be absorbed by the material during its immersion and subsequent dispersion.

A film of the present invention will exhibit its desired dispersibility when immersed in an excess of water for a time period of 15 minutes, suitably for a time period of 5 minutes, more suitably for a time period of 1 minute, and most suitably for a time period of 30 seconds.

It is been found that by using a relatively thin barrier layer and a relatively thick water-dispersible layer, a film of the present invention will exhibit effective strength and liquid impermeability properties so as to provide the desired liquid handling properties to a disposable absorbent product in which the film is being used. After use of the disposable absorbent product, the disposable absorbent product may be disposed of into an aqueous environment. When added to such an aqueous environment, the water-dispersible layer disperses into the aqueous environment. The barrier layer, being of a relatively small thickness does not unduly impede the flushability of the film and, thus, the disposable absorbent product.

In order to improve the flushability properties of the film of the present invention, it is desired that the film have an average density that is greater than about 1 gram per cubic centimeter. Such a density of the film will generally allow the film to sink to the bottom of the aqueous environment into which the film has been disposed of, such as a toilet, thereby aiding in the flushability of the film since the film will not float on the top of the water in the toilet.

The film of the present invention may generally be of any size or dimension as long as the film exhibits the desired strength and water-dispersibility properties as described herein. Generally, the film of the present invention will desirably have a thickness that is between 10 to 115 micrometers, suitably between 12 to 60 micrometers, more suitably between 12 to 40 micrometers, and most suitably between 12 to 25 micrometers.

The film of the present invention may also be used or combined with other film materials, with the film of the present invention being used as a separate layer or as an individual zone or area within a larger, composite film material. The film materials of the present invention may be combined with other film materials by methods well known to those skilled in the art, such as by using adhesives or simply by layering the different film materials together and holding together the composite materials with, for example, stitching or by application of heat and pressure.

In one embodiment of the present invention, a disposable absorbent product is provided, which disposable absorbent product comprises a liquid-permeable topsheet, a backsheet attached to the topsheet, and an absorbent structure positioned between the topsheet and the backsheet, wherein the backsheet comprises the film of the present invention.

While one embodiment of the invention will be described in terms of the use of a film material in an infant diaper, it is to be understood that the film material is equally suited for use in other disposable absorbent products known to those skilled in the art.

Fig. 1 illustrates a disposable diaper 11 according to one embodiment of the present invention. Disposable diaper 11 includes a backsheet 12, a topsheet 14, an absorbent structure 16 positioned between the backsheet 12 and the topsheet 14, wherein the backsheet 12 comprises a film of the present invention.

Those skilled in the art will recognize materials suitable for use as the topsheet and backsheet. Exemplary of materials suitable for use as the topsheet are liquid-permeable materials, such as spunbonded polypropylene or polyethylene having a basis weight of from 15 to 25 grams per square meter. Exemplary of additional materials suitable, in addition to the film of the present invention, for use as the backsheet are liquid-impervious materials, such as polyolefin films, as well as vapor-pervious materials, such as microporous polyolefin films.

Disposable absorbent products, according to all aspects of the present invention, are generally subjected during use to multiple insults of a body liquid. Accordingly, the disposable absorbent products are desirably capable of absorbing multiple insults of body liquids in quantities to which the absorbent products and structures will be exposed during use. The insults are generally separated from one another by a period of time. It is desired that when a disposable absorbent product includes the film of the present invention, the film substantially maintains its integrity or strength during use of the disposable absorbent product. As such, the amount of liquid insulting the disposable absorbent product during use should not be of such an excessive amount that the film will undergo a substantial change in integrity or strength during use of the disposable absorbent product.

After the disposable absorbent product has been used, it will be desirable to dispose of the disposable absorbent product. If the disposable absorbent product includes the film of the present invention, it may be possible to dispose of the product directly to a liquid disposal system, such as by disposing of the product into a toilet. When placed into a toilet, an excess of water will generally be present such that the water-dispersible layer of the film of the present invention may rapidly disperse into the water. The disposable absorbent product may then be capable of being flushed down the toilet without fear of the disposable absorbent product clogging the piping of the toilet. By this method of disposal, the disposable absorbent product may then be successfully treated and degraded by the sewage system to which the toilet is attached instead of disposing of the disposable absorbent product through a solid waste disposable system. By not having to be disposed of through a solid waste disposable system, the use of the film of the present invention may reduce the amount of solid waste that has to be landfilled, incinerated, or otherwise disposed of.

Test Methods

Film Liquid Transient Exposure

The Film Liquid Transient Exposure test is intended to determine if a material is liquid impermeable.

A film sample is prepared or obtained with a width of about 7.6 centimeters, a length of about 15.2 centimeters, and a thickness of about 25 micrometers, wherein the barrier layer of the film sample has a thickness of about 5 micrometers. The film sample is placed onto the raised, centrally located, rectangular surface of a base plate. The raised, centrally located, rectangular surface has a width of about 7.6 centimeters, a length of about 15.2 centimeters, and a height of about 0.3 centimeter. The base plate may be made of a flat, clear-plastic or metal plate. A liquid distribution material, having a width of about 7.0 centimeters and a length of about 14.6 centimeters, is centered on top of the film sample. The liquid distribution material is a Whatman #4 filter paper, qualitative grade, available from Whatman International Limited, Whatman Laboratory Division (catalogue number 1004 500).

A saline solution (about 2.0 milliliters) is distributed across the full width of the center of the liquid distribution material using a syringe. The saline solution is a blood bank saline solution, available from Baxter International Inc., Baxter Scientific Products Division (catalogue number B3158-3). A top plate, having dimensions similar to the base plate, is then placed over the wetted sample composite. A weight of about 4,086 grams is then placed on top of the top plate, providing a pressure of about 35.2 grams per square centimeter (about 0.5 pounds per square inch). The film sample is left alone at ambient conditions of a temperature of about 22°C and a relative humidity of 30 to 50 percent for about 60 minutes. The weight, top plate, and liquid distribution material are then removed. The film sample is then removed from the base plate. If any liquid is observed on the base plate, the film material does not pass the test and is not considered to be a material suitable for use as a barrier layer. If no liquid is observed on the base plate, the film material does pass the test and is considered to be a material suitable for use as a barrier layer.

Tensile Strength

The film sample is also evaluated for tensile strength. The tensile strength of the film sample is evaluated by using a tensile tester, such as a Model 4201 Instron with Microcon II from the Instron Corporation, Canton MA. The machine is calibrated by placing a 100 gram weight in the center of the upper jaw, perpendicular to the jaw and hanging unobstructed. The tension cell used is a 22.7 kilogram electrically-calibrating self-identifying load cell. The weight is then displayed on the Microcon display window. The procedure is performed in a room with standard-condition atmosphere such as about a temperature of about 23°C and a relative humidity of about 50 percent.

The film sample is placed in the pneumatic action grips (jaws) with 2,54 cm (1 inch) by 7,62 cm (3 inch) rubber coated (non-slipping) grip faces. The gauge length is about 15.2 centimeters, with an initial grip separation speed of about 500 millimeters per minute, and a crosshead speed of about 2000 millimeters per minute. The crosshead speed is the rate at which the upper grip moves upward pulling the film sample until failure. The tensile tester has a breaking sensitivity of about 75 percent, an extension limit of about 50.8 centimeters, and a stress limit of about 2,068 kilopascals. The tensile strength is evaluated for the film sample in both a dry condition and a wet condition, as wetted by the Film Liquid Transient Exposure test.

The Tensile Strength value is the maximum load at failure, recorded in grams of force needed to compromise or tear the sample.

$$\text{Tensile Strength = highest load at failure in N (in grams force)}$$

The Tensile Strength values of the film sample are compared in the dry and wet condition. For an ideal barrier layer, the respective Tensile Strength values should not be statistically significantly different at a 95 percent confidence level. However, a material could still experience a reduction in Tensile Strength value from a dry condition to the wet condition and still be an effective barrier layer material as long as the material did not allow any of the saline to pass through the material in the Film Liquid Transient Exposure test and the material remains essentially intact during actual use.

Examples

Example 1

Sample film materials were prepared comprising a water-dispersible layer and a potential barrier layer.

For each of the following film samples, the prepared film had a thickness of about 25 micrometers. The water-dispersible layer comprised about 80 weight percent, or about 20 micrometers, of the film sample. The potential barrier layer comprised about 20 weight percent, or about 5 micrometers, of the film sample.

For each of the samples, the water-dispersible layer comprised a mixture of about 80 weight percent of polyethylene oxide, having a molecular weight of about 200,000 and a viscosity range as a 5 weight percent aqueous solution at about 25°C of 0,65 to 1,15 Pa·s (65 to 115 centipoise), commercially available from the Union Carbide Company under the trade designation Polyox WSR N-80 water-soluble resin, and about 20 weight percent of poly(ethylene-co-acrylic acid), having an acrylic acid comonomer amount of about 9.5 weight percent, available from The Dow Chemical Company under the designation Primacor 1410 poly(ethylene-co-acrylic acid). The polyethylene oxide and poly(ethylene-co-acrylic acid) were mixed in an extruder and coextruded with the barrier layer into a film of the desired thickness.

For Sample 1, the potential barrier layer comprised poly(ethylene-co-acrylic acid), having an acrylic acid comonomer amount of about 9.5 weight percent, available from The Dow Chemical Company under the designation Primacor 1430 poly(ethylene-co-acrylic acid). The poly(ethylene-co-acrylic acid) was mixed in an extruder and coextruded with the water-dispersible layer into a film of the desired thickness.

For Sample 2, the potential barrier layer comprised a mixture of about 10 weight percent of polyethylene oxide, having a molecular weight of about 200,000 and a viscosity range as a 5 weight percent aqueous solution at about 25°C of 0,65 to 1,15 Pa·s (65 to 115 centipoise), commercially available from the Union Carbide Company under the trade designation Polyox WSR N-80 water-soluble resin, and about 90 weight percent of poly(ethylene-co-acrylic acid), having an acrylic acid comonomer amount of about 9.5 weight percent, available from The Dow Chemical Company under the designation Primacor 1410 poly(ethylene-co-acrylic acid). The polyethylene oxide and poly(ethylene-co-acrylic acid) were mixed in an extruder and coextruded with the water-dispersible layer into a film of the desired thickness.

For Sample 3, the potential barrier layer comprised a mixture of about 20 weight percent of polyethylene oxide, having a molecular weight of about 200,000 and a viscosity range as a 5 weight percent aqueous solution at about 25°C of 0,65 to 1,15 Pa·s (65 to 115 centipoise), commercially available from the Union Carbide Company under the trade designation Polyox WSR N-80 water-soluble resin, and about 80 weight percent of poly(ethylene-co-acrylic acid), having an acrylic acid comonomer amount of about 9.5 weight percent, available from The Dow Chemical Company under the designation Primacor 1410 poly(ethylene-co-acrylic acid). The polyethylene oxide and poly(ethylene-co-acrylic acid) were mixed in an extruder and coextruded with the water-dispersible layer into a film of the desired thickness.

For Sample 4, the potential barrier layer comprised a polylactic acid, having a molecular weight of about 70,000, available from the Cargill Company under the designation EcoPla 520 polylactic acid. The polylactic acid was mixed in an extruder and coextruded with the water-dispersible layer into a film of the desired thickness.

The film samples were then evaluated according to the Film Liquid Transient Exposure and Tensile Strength tests described herein. The results are summarized in Table 1.

TABLE 1

| Sample No. | Tensile Strength N (grams force) | | Acceptable Barrier Layer ? |
|---|---|---|---|
| | Dry | Wet | |
| 1 | 4,903 (4903) | 4,994 (4994) | Yes |
| 2 | 5,720 (5720) | 4,630 (4630) | Yes |
| 3* | 5,039 (5039) | 1,438 (1498) | No |
| 4 | 5,084 (5084) | 4,857 (4857) | Yes |

* Not an example of the present invention.

Example 2

The Sample 1 film prepared in Example 1 was laminated to a coform absorbent containing 30 weight percent meltblown polymer and 70 weight percent fluff pulp with an overall basis weight of about 190 grams per square meter, and then converted into a pantiliner on a production machine. Control pantiliners were also prepared using a polyethylene film as the barrier layer.

The pantiliners were placed in a consumer study with adult women. The Sample 1 film was found to be capable as acting as an effective backsheet by preventing any leakage of small liquid loads to the pantiliners from urine and menses for periods of up to six hours.

Twenty samples each of both of the pantiliners were subjected to a toilet flushing test. In the test, individual pantiliner samples were placed at random in a 13,25 l (3.5 gallon) toilet and were allowed to dwell in the toilet for about 30 seconds before flushing. The control pantiliners only flushed in six out of the twenty samples. In contrast, all twenty samples for the Sample 1 film of the present invention flushed. A visual observation that was made during while conducting this test was that the pantiliners according to the present invention dispersed almost immediately and sank directly to the bottom of the toilet bowl. In contrast, the control pantiliners, which contained polyethylene baffle film (which had a density less than 1 gram per cubic centimeter) floated on the surface of the water in the toilet bowl. Consequently, the hydraulic driving force acting on the control product was much less than that acting on the experimental products. This demonstrated the lack of flushability of the control product because it could not realize the driving force of the priming jet in the toilet.

Those skilled in the art will recognize that the present invention is capable of many modifications and variations without departing from the scope thereof. Accordingly, the detailed description and examples set forth above are meant to be illustrative only and are not intended to limit, in any manner, the scope of the invention as set forth in the appended claims.

**Claims**

1. A film comprising a liquid barrier layer that has a thickness between 2 to 15 micrometers attached to a water-dispersible layer that has a thickness between 10 to 100 micrometers and comprises 55 to 95 wt.-% polyethylene oxide and 45 to 5 wt.-% poly(ethylene-co-acrylic acid), based on the total amount of polyethylene oxide and poly(ethylene-co-acrylic acid) present in said water-dispersible layer, and wherein the film has an average density greater than about 1 gram per cubic centimeter.

2. The film of claim 1 wherein the water-dispersible layer has a thickness between 10 to 50 micrometers.

3. The film of claim 1 or 2 wherein the barrier layer comprises acrylic acid copolymer, polylactic acid, maleic anhydride modified polyethylene, poly(ethylene-co-acrylic acid), polycaprolactone, polyester, a polyhydroxyalkanoate, copolymers or mixtures thereof.

4. The film according to any one of claims 1 to 3 wherein the barrier layer has a thickness between 2 to 10 micrometers.

5. The film according to any one of the preceding claims wherein the film is prepared by an extrusion process.

6. A disposable absorbent product comprising a liquid-permeable topsheet, a backsheet attached to the liquid-permeable topsheet, and an absorbent structure positioned between the liquid-permeable topsheet and the backsheet, wherein the backsheet comprises a film according to any one of claims 1 to 5.

**Patentansprüche**

1. Folie umfassend eine Flüssigkeitssperrlage mit einer Dicke zwischen 2 und 15 Mikrometern, die an einer wasserdispergierbaren Lage mit einer Dicke zwischen 10 und 100 Mikrometern angebracht ist, die 55 bis 95 Gewichtsprozent Polyethylenoxid und 45 bis 5 Gewichtsprozent Poly(ethylen-co-acrylsäure) umfasst, basierend auf dem Gesamtanteil des in der wasserdispergierbaren Lage vorhandenen Polyethylenoxids und der Poly(ethylen-co-acrylsäure), und wobei die Folie eine durchschnittliche Dichte von mehr als etwa 1 Gramm pro Kubikzentimeter aufweist.

2. Folie gemäß Anspruch 1, wobei die wasserdispergierbare Lage eine Dicke zwischen 10 und 50 Mikrometer aufweist.

3. Folie gemäß Anspruch 1 oder 2, wobei die Sperrlage Acrylsäurecopolymer, Polymilchsäure, maleinsäureanhydridmodifiziertes Polyethylen, Poly(ethylen-co-acrylsäure), Polycaprolacton, Polyester, ein Polyhydroxyalkanoat, Copolymere oder Gemische davon umfasst.

4. Folie gemäß einem der Ansprüche 1 bis 3, wobei die Sperrlage eine Dicke zwischen 2 und 10 Mikrometer aufweist.

5. Folie gemäß einem der vorhergehenden Ansprüche, wobei die Folie durch ein Extrusionsverfahren hergestellt wurde.

**6.** Absorbierendes Wegwerfprodukt umfassend eine flüssigkeitsdurchlässige Vorderschicht, eine an der flüssigkeitsdurchlässigen Vorderschicht angebrachte Rückschicht und eine zwischen der flüssigkeitsdurchlässigen Vorderschicht und der Rückschicht angeordnete absorbierende Struktur, wobei die Rückschicht eine Folie gemäß einem der Ansprüche 1 bis 5 umfasst.

## Revendications

**1.** Film constitué d'une couche formant barrière aux liquides, qui a une épaisseur comprise entre 2 et 15 µm, fixée à une couche dispersable dans l'eau ayant une épaisseur comprise entre 10 et 100 µm et constituée de 55 à 95% en poids de poly(oxyde d'éthylène) et de 45 à 5% en poids de poly(éthylène-co-acide acrylique), par rapport au poids total de poly(oxyde d'éthylène) et de poly(éthylène-co-acide acrylique) présent au sein de ladite couche dispersable dans l'eau, le film ayant une masse spécifique moyenne supérieure à environ 1 g/cm$^3$.

**2.** Film selon la revendication 1, dans lequel la couche dispersable dans l'eau a une epaisseur comprise entre 10 et 50 µm.

**3.** Film selon la revendication 1 ou 2, dans lequel la couche formant barrière est constituée d'un copolymère d'acide acrylique, d'un poly(acide lactique), d'un polyéthylène modifié par l'anhydride maléique, d'un poly(éthylène-co- acide acrylique), d'une polycaprolactone, d'un polyester, d'un polyhydroxyalcanoate, de copolymères ou mélanges de ceux-ci.

**4.** Film selon l'une quelconque des revendications 1 à 3, dans lequel la couche formant barrière a une épaisseur comprise entre 2 et 10 µm.

**5.** Film selon l'une quelconque des revendications précédentes, dans lequel le film est préparé par un procédé d'extrusion.

**6.** Produit absorbant jetable constitué d'une feuille supérieure perméable aux liquides, d'une feuille support fixée à la feuille supérieure perméable aux liquides, et d'une structure absorbante disposée entre la feuille supérieure perméable aux liquides et la feuille support, produit dans lequel la feuille support est constituée d'un film selon l'une quelconque des revendications 1 à 5.

FIG. 1